# EUROPEAN PATENT APPLICATION

(11) **EP 1 127 558 A1**
(43) Date of publication of application: **29.08.2001**
(21) Application number: 00200565.0
(22) Date of filing: 18.02.2000
(51) Int. Cl.: A61F 2/30, A61F 2/46

(54) **Plug for insertion into a bone canal**

(71) Applicant: IsoTis N.V., 3723 MB Bilthoven (NL)
(72) Inventor: The designation of the inventor has not yet been filed
(74) Representative: Ottevangers, Sietse Ulbe

(57) **Abstract**

Plug (1) comprising a carrier (2) carrying first (5) and second (6) flexible flanges for cooperating with the wall (25) of a bone canal (22), each flange (5,6) extends radially outwardly relative to the longitudinal axis (A) of the carrier (2). The plug (1) further comprises means for axially moving at least one (6) of said flexible flanges along the axis (A) of the carrier (2), such that, when the plug (1) is inserted in a bone canal (22), the axial distance (4) between radial inner portions (8) of the flanges (5,6) can be decreased.

## Description

The invention relates to a plug, comprising an elongate carrier carrying first and second flexible flanges for cooperating with the walls of a bone canal, each flange extending radially outwardly relative to the longitudinal axis of the carrier.

Such a plug is generally known and is used to restrict the flow of cement during prosthesis implantation.

During implant surgery and in particular during implantation of a hip prosthesis, it is known to cement the prosthesis to the bone. In order to cement the prosthesis to the bone, the bone canal is broached or reamed, such that the trabecular, porous bone portions are removed and the remaining cortical, hard bone portions define the walls of the bone canal. An anchoring portion of the prosthesis is then inserted into the bone canal, for example an anchoring portion of a hip prothesis is inserted into a broached femural bone, such that a ball portion extends outward to replace the natural ball portion of the hip bone.

In order to fix the anchoring portion of the prosthesis to the bone, cement, such as polymethylmethacrylate (PMMA) is inserted into the bone canal and is packed upon the prosthesis prior to insertion thereof. This cement then anchors the anchoring portion of the prosthesis to the bone material.

In order to provide for a secure joint between the prosthesis and the bone at the cement interface, it is desired to have the cement completely surround the prosthesis in the interstices between the prosthesis and the bone material. However, the insertion forces required to insert the implant often drive the cement substance down into the intramedullary canal and away from the fixation area. This way, voids are formed in the cement mantle which later become stress points leading to early fatigue of the prothesis and/or the fixation.

In order to restrict the flow of bone cement further into the intramedullary canal, intramedullary plugging devices such as plugs have been developed which can be inserted into the bone canal as a blockage. Usually, the plug is provided with a number of fins or other flange type projections. When the implant is forced into the broached portion of the intramedullary canal, the tendency of the restricted bone cement to flow down the canal is prevented by the fins of the plug cooperating with the walls of the channel.

A problem associated with the known plugs is that the flanges do not always cooperate sufficiently sealingly or blockingly with the bone channel. This is caused by the fact that the bone channel as defined by the cortical bone has a cross-section that varies along its longitudinal axis. In particular, the cross-section is of substantially elliptical or oval shape at the onset of the channel and axially inwardly becomes more circularly shaped at a midportion. Further inwardly, the cross-section becomes substantially oval or elliptically shaped again, having an orientation that is rotated around the axis of the canal to a position wherein it is substantially perpendicular to the orientation of the first section. In addition, for each person, the size of the bone and therewith the cross-section and axial dimension of the bone channel varies.

During implantation, the plug has to be placed sealingly at a predetermined location within the bone canal, e.g. about 1.5 cm axially inward from the preferred location of the lower tip of the anchoring portion after insertion.

Due to the varying shape of the bone canal it has proven difficult to provide a universal plug that can be used to plug any channel at any axial location. Depending on the circumstances, a good chance exists that cement can either pass the flanges of the plug or the plug can escape inwardly into the channel upon insertion of the anchoring portion. Alternatively, it may not be possible to fix the plug at the desired location, because it is either too large or too small. Although it has been proposed to provide a set of plugs, each having flanges of different sizes, selecting the right plug has proven a problem. This problem is particularly relevant, since the assessment of the plug size is to be made prior to insertion. In addition, the costs of storage and production of such a set are considerable.

The invention aims to provide a plug of the aforementioned type in which the above problem is overcome. According to the invention, this problem is solved by providing means for axially moving at least one of said flexible flanges along the axis of the carrier, such that, when the plug has been inserted in a bone canal, the axial distance between radial inner portions of the flanges located near the axis of the carrier can be decreased. This way, the plug can be inserted into the bone canal, while both flanges flex to assume a forwardly oriented cup-like shape, until the second flexible flange is at the desired location and the first flange is axially spaced therefrom in inward direction. By subsequently retracting the first flange to a second position wherein the flanges are axially closer, the orientation of the first flexible flange can be flipped to a cup-shape having an opposite orientation and the first and second flanges of the plug exhibit a substantially diabolo-like configuration. This way, the second, axially upper flange constitutes a conventional seal that is placed at the desired location, while the first, axially lower flange provides a dome-like support structure guiding axial forces towards the walls of the bone canal. In addition, the first flange may act as a secondary seal.

The moving means for axially moving at least one of the flexible flanges along the axis of the carrier, can be designed in many ways.

It is e.g. possible to mount one of the flanges slidingly onto a shaft-like portion of the carrier by means of a hole or channel, while the other flange is fixedly connected to the carrier. Furthermore, the moving means can be embodied as screw thread on which a flange is mounted onto the carrier, or similar means for transforming rotation of the shaft into axial translation of the flange. By mounting two flanges on screw threads having opposite directions, rotation of the carrier can cause the flanges to simultaneously move axially closer in the bone canal. In addition, the moving means can be realized by providing telescopic movement of two co-axial axes, e.g. an inner axis carrying the first flange and an outer axis carrying the second flange.

Such and further embodiments for performing the above function all lie within the scope of the invention.

Preferably, the flanges are substantially circular to improve interaction with the bone channel. By providing cuts or folds extending inwardly from the outer peripheral portion, interaction with the canal walls can be improved further. In a preferred embodiment the flanges are thin walled and/or have an outer peripheral portion of reduced thickness for improving flexibility.

In another embodiment, the carrier comprises separation means, such that at least a part of the carrier can be removed from the flanges. This way, after placement of the plug, the remaining volume of the plug into the canal can be minimized. Preferably, the separation means are embodied as a breakable connection having reduced tensile strength and/or torsional strength.

In yet another embodiment, the flanges and/or the carrier are made from a bio-compatible and/or bio-degradable material. Preferably, the carrier and/or flanges are made from a swellable material, such that after insertion and upon contact with fluid, the sealing and locking closure of the plug in the bone canal can be improved further.

In the context of the present invention, the term biocompatible is intended to refer to materials which may be incorporated into a human or animal body, e.g. in the form of a medical implant, substantially without unacceptable responses of the human or animal. The term biodegradable refers to materials which, after a certain period of time, are broken down in a biological environment. Preferably, the rate of breakdown is chosen similar or identical to the rate at which the body generates autogenous tissue to replace an implant, such as the present plug, of which the biodegradable material is manufactured.

A specific class of swellable materials, which has been found to lead to particularly good results, is the class of copolymers of a polyalkylene glycol and an aromatic polyester. Preferably, these copolymers comprise 40-80 wt.%, more preferably 50-70 wt.% of the polyalkylene glycol, and 60-20 wt.%, more preferably 50-30 wt.% of the aromatic polyester. A preferred type of copolymers according to the invention is formed by the group of block copolymers.

Preferably, the polyalkylene glycol has a weight average molecular weight of from 150 to 4000, more preferably of 200 to 1500. The aromatic polyester preferably has a weight average molecular weight of from 200 to 5000, more preferably of from 250 to 4000. The weight average molecular weight of the copolymer preferably lies between 20,000 and 200,000, more preferably between 50,000 and 120,000. The weight average molecular weight may suitably be determined by gel permeation chromatography (GPC). This technique, which is known per se, may for instance be performed using tetrahydrofuran as a solvent and polystyrene as external standard.

In a preferred embodiment, the polyalkylene glycol component has units of the formula -OLO-CO-Q-CO-, wherein O represents oxygen, C represents carbon, L is a divalent organic radical remaining after removal of terminal hydroxyl groups from a poly(oxyalkylene)glycol, and Q is a divalent organic radical.

Preferred polyalkylene glycols are chosen from the group of polyethylene glycol, polypropylene glycol, and polybutylene glycol and copolymers thereof, such as poloxamers. A highly preferred polyalkylene glycol is polyethylene glycol.

The terms alkylene and polyalkylene generally refer to any isomeric structure, i.e. propylene comprises both 1,2-propylene and 1,3-propylene, butylene comprises 1,2-butylene, 1,3-butylene, 2,3-butylene, 1,2-isobutylene, 1,3-isobutylene and 1,4-isobutylene (tetramethylene) and similarly for higher alkylene homologues. The polyalkylene glycol component is preferably terminated with a dicarboxylic acid residue -CO-Q-CO-, if necessary to provide a coupling to the polyester component. Group Q may be an aromatic group having the same definition as R, or may be an aliphatic group such as ethylene, propylene, butylene and the like.

The polyester component preferably has units -O-E-O-CO-R-CO-, wherein O represents oxygen, C represents carbon, E is a substituted or unsubstituted alkylene or oxydialkylene radical having from 2 to 8 carbon atoms, and R is a substituted or unsubstituted divalent aromatic radical.

In a preferred embodiment, the polyester is chosen from the group of polyethylene terephtalate, polypropylene terephtalate, and polybutylene terephtalate. A highly preferred polyester is polybutylene terephtalate.

The preparation of the copolymer will now be explained by way of example for a polyethylene glycol/polybutylene terephtalate copolymer. Based on this description, the skilled person will be able to prepare any desired copolymer within the above described class. An alternative manner for preparing polyalkylene glycol/polyester copolymers is disclosed in US-A-3,908,201.

A polyethylene glycol/polybutylene terephtalate copolymer may be synthesized from a mixture of dimethyl terephtalate, butanediol (in excess), polyethylene glycol, an antioxidant and a catalyst. The mixture is placed in a reaction vessel and heated to about 180°C, and methanol is distilled as transesterification proceeds. During the transesterification, the ester bond with methyl is replaced with an ester bond with butylene. In this step the polyethyene glycol substantially does not react. After transesterification, the temperature is raised slowly to about 245°C, and a vacuum (finally less than 0.1 mbar) is achieved. The excess butanediol is distilled and a prepolymer of butanediol terephtalate condenses with the polyethylene glycol to form a polyethylene/polybutylene terephtalate copolymer. A terephtalate moiety connects the polyethylene glycol units to the polybutylene terephtalate units of the copolymer and thus such copolymer also is sometimes referred to as a polyethylene glycol terephtalate/polybutylene terephtalate copolymer (PEGT/PBT copolymer).

The invention also relates to a method of inserting a plug into a bone canal.

Further preferred embodiments of the invention are described in the appended claims.

The invention will be elucidated further by means of a drawing. In the drawing is:
Fig. 1 a front view of a plug according to the invention, the flanges being partially cut away to enhance clarity;
fig. 2 a perspective view of the plug of fig. 1;
fig. 3 a cross-section of a femur having a reamed bone canal;
fig. 4 - fig. 8 a set of diagrams showing consecutive steps of insertion of the plug; and
fig. 9 a cross-section of the femur of fig. 3 with the plug in situ and a prothesis cemented into the bone canal.

The drawings are schematic representations of preferred embodiments. In the drawings, like or corresponding parts are identified with the same reference numerals.

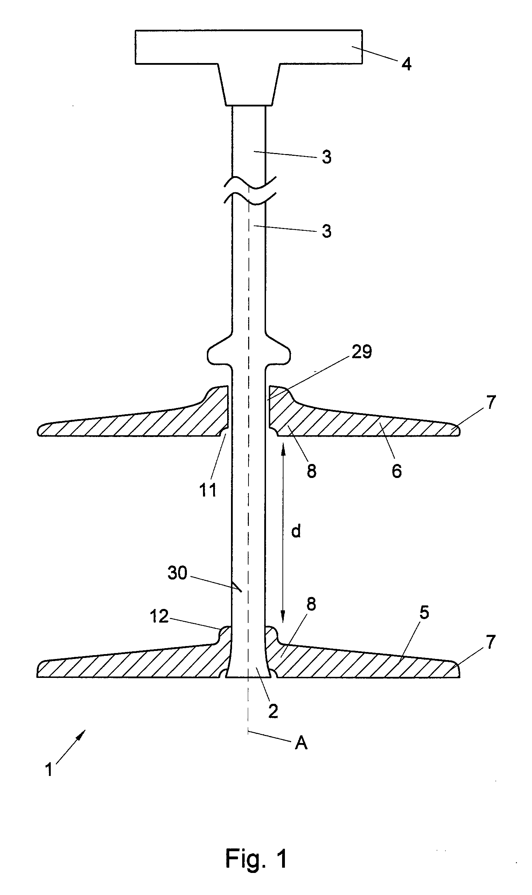

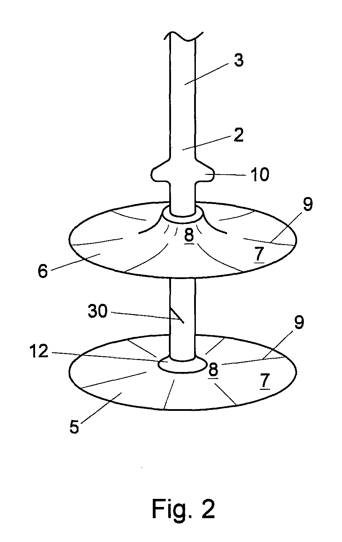

Figures 1 and 2 show a plug 1 comprising an elongate carrier 2 having a longitudinal axis A.

The elongate carrier 2 is integrally formed with a shaft assembly 3 carrying a handle 4.

The carrier 2 carries a first and a second flexible flange 5, 6 respectively. Each flange 5, 6 extends radially outwardly relative to the longitudinal axis A of the carrier 2. The flanges 5, 6 are thin walled, i.e. their dimension in the direction of axis A is small relative to the dimension to which they extend radially outward relative to the axis A. By providing an outer peripheral portion 7 of a flange 5, 6 with a thickness T that is smaller than the thickness of an inner portion 8 close to the carrier 2, the flexibility of the flange 5, 6 can be increased. To enhance the ability of the outer peripheral portions 7 of the flanges 5, 6 to conform to the inner shape of the bone canal into which the plug 1 is to be inserted, the outer peripheral portion 7 of the flanges can be provided with radially extending cuts 9. In this embodiment, the flanges 5, 6 are preferably of circular shape and of identical size. The flanges 5, 6 are of such size that their axial projection onto the bone canal is larger than the cross-section of the bone canal, such that upon insertion, peripheral portions of the flanges can engage the walls of the bone canal.

The moving means are embodied as follows. The first flange 5 is axially fixed to the carrier 2, while the second flange 6 can slide along the longitudinal axis A of a shaft like portion of the carrier 2 by means of a hole 29. The sliding movement of the second flange 6 is limited in axial direction by the first flange 5 and a stop boss 10 respectively; such that the axial distance d of the radial inner portions 8 of the flanges 5, 6 located close to the carrier 2 can be varied.

The flanges 5, 6 are provided with cooperating connecting means on their respective radial inner portions 8, which connecting means cooperate when the inner portions 8 of the flanges 5, 6 are in abutment to interlock the flanges 5, 6 and to prevent passage of cement. The cooperating connecting means are here embodied as a conical ridge 11 on the inner portion 8 of the first flange 5 cooperating with a correspondingly formed depression 12 of slightly smaller dimensions located in the inner portion 8 of the second flange 6. It shall be clear that the cooperating connecting means can be designed in may ways, e.g. as snap-fingers.

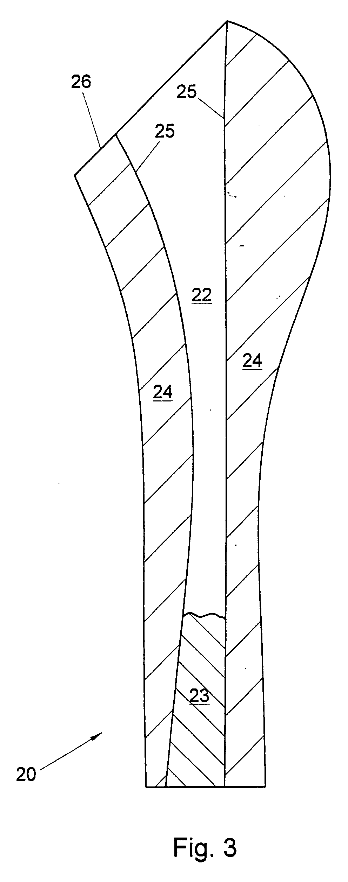

The insertion of the plug 1 into the bone canal is now shown with reference to Figures 3-10.

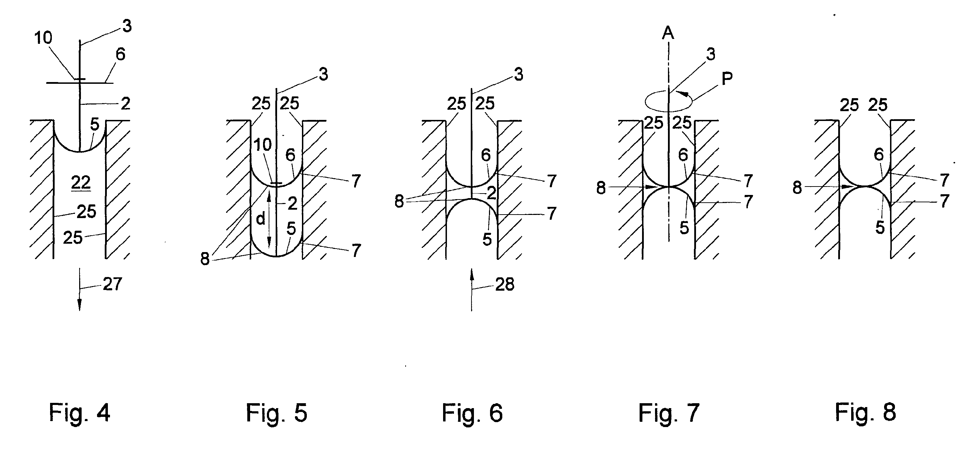

Figure 3 shows a femural bone 20 that has been prepared for implantation of a hip prosthesis 21. A bone canal 22 is reamed by removing the porous trabecular bone 23, such that the hard, cortical bone defines the walls 25 of the bone canal 22. As is shown in Figures 4-8, the plug 1 is inserted into the bone canal 22. Upon insertion of the plug 1 by moving the shaft assembly 3 in the direction of arrow 27 inwards into the bone canal 22, firstly the first flange 5 flexes backwardly into a cup-shaped orientation (Fig. 4). Upon deeper insertion, the second flexible flange 6 is retained by the stop boss 10 and is entrained into the bone canal 22 and flexes backwardly into a cup-shape having the same orientation as the first flange 5. The outer peripheral portions 7 of the flanges 5, 6 cooperate with the walls 25 of the bone canal 22. When the second flange 6 has reached the desired location in the bone canal 22 the inserting movement is stopped. By providing a scale on the shaft assembly 3, the correct depth of insertion of the plug into the canal 22 relative to the edge 26 of the bone can be ensured. The fully inserted position is shown in Figure 5.

Subsequently, the axial distance d between the radial inner portions 8 of the flanges 5, 6 is decreased. In this embodiment, the elongate carrier 2 is retracted in the direction of arrow 28 by pulling the handle 4 of the shaft assembly 3 outward. The carrier 2, which is fixedly connected to the first flange 5, entrains first flange 5 upward while the second flange 6, which is slidably mounted onto the carrier 2 via opening 29, remains stationary in the bone canal 22. The first flange 5 now flips its orientation because the inner peripheral portion 8 is entrained with the carrier 2, while the outer peripheral portion 7 is retained by the channel walls 25 until the first flange 5 has a cup-shaped orientation opposite to the cup-shaped orientation of the second flange 6. The plug 1 is now substantially diabolo-shaped, as is shown in Figure 6. Upon further retraction of the shaft assembly 3 and the elongate carrier 2, the outer peripheral portion 7 of the second flexible flange 6 is entrained along the channels 25 of the bone canal 22 until the inner portions 8 of the flanges 5, 6 are in abutment as shown in Figure 7. In this position, the cooperating connecting means, if present, can interlock, e.g. the ridge 11 of the first flange 5 is pulled into the depression 12 of the second flange 6 to form a fluid tight barrier.

By twisting the shaft assembly 3 in the direction of arrow P around its axis A, a breakable connection 30 between the carrier 2 and the shaft assembly 3 can be severed and the shaft assembly 3 and/or a portion of the elongate carrier 2 can be subsequently removed. Preferably, the breakable connection 30 is embodied as a radial inwardly extending notch which reduces torsional resistance of the shaft assembly 3 by providing a starting point for torsional shear, while leaving sufficient tensile and compressional strength. The result of breaking the breakable connection is shown in Figure 8. In this position, the plug is fully applied. The second flange 6 provides a first seal for blocking passage of cement 31 into the bone canal 22 in the direction of arrow 25, while the first flange 5 serves as a dome-shaped support for transmitting forces associated with axially inward pressure of the cement 31 and/or prosthesis 21 towards the walls 25 of the bone canal 22.

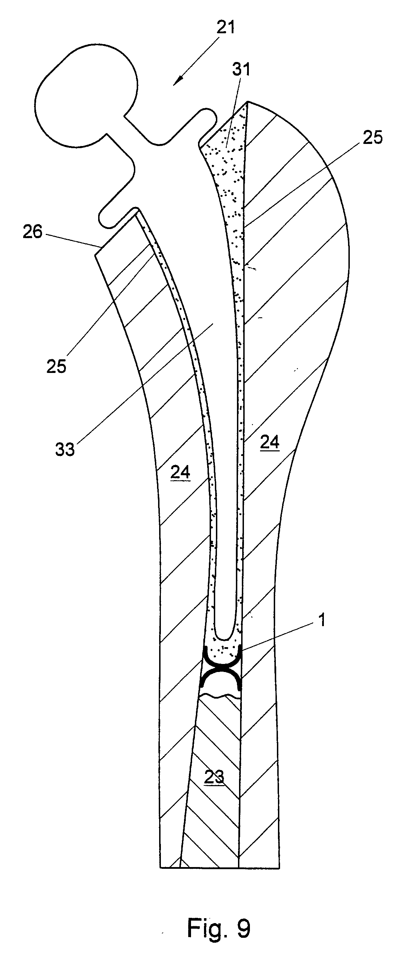

Subsequently, the prosthesis 21 is inserted into the bone canal 22 with its anchoring portion 33 packed with cement 31 to obtain the situation as shown in Figure 9.

It shall be clear to the skilled man that the invention is not limited to the above-described preferred embodiment and that many variations are possible. In particular, the plug may comprise more than two flanges and the flanges may (each) be of different sizes or shapes, e.g. oval shapes and/or shapes that are asymmatrical and/or biased towards one or more orientations. Furthermore, the means for adjusting the axial distance between the flanges can be constructed differently. The flanges can, depending on the embodiment chosen, be inserted into the bone canal to become axially spaced or the flanges can be axially spaced prior to insertion. Also, the means for detaching the plug 1 from the shaft assembly 3 can be constructed differently, e.g. by a bajonet-type closure or a screw-thread.

Such variations will readily spring to the mind of the skilled man and are considered to be within the scope of the present invention as defined in the appended claims.

## Claims

1. Plug, comprising a carrier carrying first and second flexible flanges for cooperating with the walls of a bone canal, each flange extending radially outwardly relative to the longitudinal axis of the carrier, and means for axially moving at least one of said flexible flanges along the axis of the carrier, such that, when the plug has been inserted in a bone canal, the axial distance between radial inner portions of the flanges located near the axis of the carrier can be decreased.

2. Plug according to claim 1, wherein the flanges are substantially circular.

3. Plug according to claims 1 or 2, wherein at least one of the flanges comprises radially extending cuts and/or folds in an outer circumferential portion.

4. Plug according to any of the preceding claims, wherein the flanges have an outer peripheral portion of reduced thickness for improving flexibility.

5. Plug according to any of the preceding claims, wherein at least of the flanges is substantially planar prior to insertion.

6. Plug according to any of the preceding claims, wherein the plug comprises separation means, such that a shaft-assembly or at least a part of the carrier can be removed.

7. Plug according to any of the preceding claims, wherein the first and second flexible flanges comprise cooperating connecting means to interlock the flanges when their inner portions are in abutment.

8. Plug according to any of the preceding claims, wherein the flanges and/or the carrier are made from a swellable material.

9. Plug according to any of the preceding claims, wherein the flanges and/or the carrier are made from a bio-compatible and/or bio-degradable material.

10. Method of inserting a plug into a bone canal, comprising the steps of inserting a carrier carrying first and second flexible flanges into a bone canal, such that the flanges cooperate with the walls of the bone canal to assume inwardly oriented, parallel cup-like shapes, and wherein the first flange is subsequently axially retracted relative to the second flange to a position wherein radial inner portions of the flanges are axially closer, such that the orientation of the first flexible flange is flipped to a cup-shape having an opposite orientation and the first and second flanges of the plug exhibit a substantially diabolo-like configuration.

11. Method according to claim 10, wherein subsequently a shaft assembly or a portion of the carrier carrying the flanges is disconnected and is axially retracted from the bone canal.
